# EUROPEAN PATENT SPECIFICATION

(11) **EP 2 060 225 B1**
(45) Date of publication and mention of the grant of the patent: **15.01.2014**
(21) Application number: 08253690.5
(22) Date of filing: 11.11.2008
(51) Int. Cl.: A61B 1/005, A61B 17/00, A61L 29/14, A61M 25/00

(54) **System for rigidizing flexible medical implements**
System zum Versteifen von flexiblen medizinischen Instrumenten
Système pour rigidifier des instruments médicaux flexibles

(30) Priority: 13.11.2007 US 987453 P; 10.11.2008 US 267752
(43) Date of publication of application: 20.05.2009
(73) Proprietor: Covidien LP, Mansfield, MA 02048 (US)
(72) Inventor: Viola, Frank J., Sandy Hook, CT 06482 (US)
(74) Representative: Soames, Candida Jane

(56) References cited:
- WO-A-02/096276
- DE-A1- 3 039 551
- DE-A1- 3 707 787
- JP-A- 63 305 836
- US-A- 4 176 662
- US-A- 5 759 151

## Description

### BACKGROUND

### 1. Technical Field

The present disclosure relates generally to flexible steerable instruments, such as steerable catheters and/or probes which are remotely operated in endoscopic, endoluminal and laparoscopic surgical procedures. In particular, the present disclosure is directed to a system and method for rigidizing a flexible steerable instrument.

### 2. Background of Related Art

Various minimally invasive surgical procedures utilize endoscopic, endoluminal and laparoscopic surgical techniques. These techniques generally involve insertion of surgical instruments through small incisions. One example of such surgical instruments is a flexible steerable instrument which may have a tool assembly (e.g., grasping jaws, cutting tool, camera, suction attachment, etc.) attached at a distal end of the instrument. These instruments can be navigated and steered inside the patient's body due to their flexibility. Once in position, it is often desired for the flexible steerable instruments to be held in a particular position to perform the desired tissue manipulation using the tool assembly.

US 5759151 addresses this problem by providing a flexible sheath with a stiffening controller.

Conventional flexible steerable instruments include two or more segments which are configured to pivot and/or swivel relative to each other by using one or more tensile elements running therethrough. The tensile elements are coupled to the distal segment and when the elements are tightened (e.g., pulled in the proximal direction) the segments are drawn together preventing the segments from sliding and/or pivoting due to friction forces between the segments thereby rigidizing the flexible instrument. One drawback of the conventional flexible steerable instruments utilizing tensile elements is that if an insufficient amount of tension is provided in the tensile elements, the steerable instrument may continue to swivel and/or pivot upon application of a force to the instrument. Therefore there is a need for a novel flexible steerable instrument configured to maintain its rigidity.

### SUMMARY

According to the present invention a flexible steerable device is disclosed. The flexible steerable device includes a plurality of interconnected segments configured to pivot relative to each other, each of the plurality of segments having a cavity disposed therein. The device also includes a flexible tube disposed within the cavities of the plurality of segments. The flexible tube includes at least one rigidizing chamber having fusible material disposed therein, wherein upon varying the temperature of the fusible material, the fusible material shifts between a first state in which the flexible steerable device is flexible and a second state in which the flexible steerable device is rigidized.

According to another aspect of the present disclosure a flexible steerable device also includes a plurality of fittings disposed between each of the plurality of interconnected segments, each of the plurality of fittings including a flexible membrane and fusible material disposed therein.

A method for rigidizing a flexible steerable device is also contemplated by the present disclosure. The method includes the steps of providing a flexible steerable device having a plurality of interconnected segments configured to pivot relative to each other, each of the plurality of segments having a cavity disposed therein and a flexible tube disposed within the cavities of the plurality of segments. The flexible tube includes at least one rigidizing chamber having a heating element and fusible material disposed therein. The method also includes the steps of increasing heat applied to the fusible material thereby liquefying the fusible material and decreasing heat applied to the fusible material thereby solidifying the fusible material and rigidizing the flexible tube and the plurality of interconnected segments.

### BRIEF DESCRIPTION OF THE DRAWINGS

The above and other aspects, features, and advantages of the present disclosure will become more apparent in light of the following detailed description when taken in conjunction with the accompanying drawings in which:
Fig. 1 is a perspective view of a flexible steerable instrument according to one embodiment of the present disclosure;
Fig. 2 is a side cross-sectional view of a segment of the flexible steerable instrument according to one embodiment of the present disclosure;
Fig. 3 is a perspective view of a section of a flexible tube of the flexible steerable instrument according to one embodiment of the present disclosure;
Fig. 4 is a side cross-sectional view of the flexible steerable instrument according to one embodiment of the present disclosure;
Fig. 5 is a perspective view with parts separated of the flexible steerable instrument according to one embodiment of the present disclosure;
Figs. 6 and 7 are side cross-sectional view of the flexible steerable instrument according to one embodiment of the present disclosure;
Fig. 8 is a perspective view with parts separated of the flexible steerable instrument according to another embodiment of the present disclosure;
Fig. 9 is a side cross-sectional view of a membrane of the flexible steerable instrument according to another embodiment of the present disclosure;
Fig. 10 is a perspective view of a section of a flexible tube of the flexible steerable instrument according to another embodiment of the present disclosure;
Figs. 11 and 12 are side cross-sectional view of the flexible steerable instrument according to another embodiment of the present disclosure;
Fig. 13 is a side view of the flexible steerable instrument according to another embodiment of the present disclosure; and
Fig. 14 is a side view of the flexible steerable instrument according to another embodiment of the present disclosure.

### DETAILED DESCRIPTION

Embodiments of the present disclosure will be described herein below with reference to the accompanying drawings. In the following description, well-known functions or constructions are not described in detail to avoid obscuring the present disclosure in unnecessary detail.

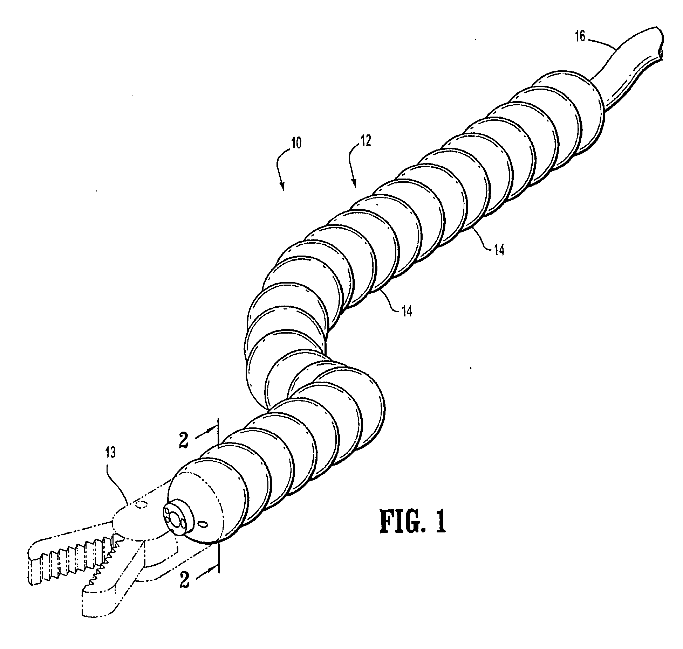

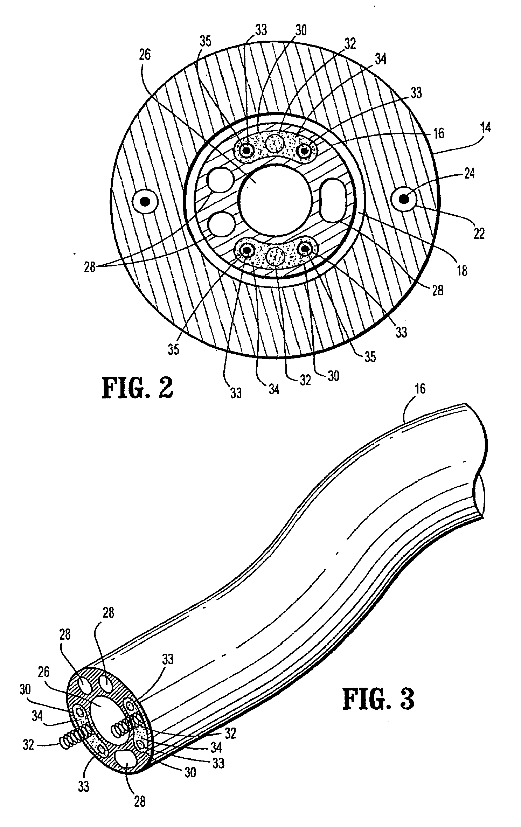

Referring to Fig. 1, a flexible steerable instrument 10 is shown. The instrument 10 includes an elongated flexible body 12 and a tool assembly 13 disposed at the distal end of the body 12. In embodiments, the tool assembly 13 may be a pair of opposing jaws as shown, e.g., graspers, pliers, etc., or other surgical tools, such as a cutting tool, e.g., scissors, dissectors, etc., and the like. Although not shown, it is envisioned that the presently disclosed instrument could be easily adapted for RF or electrical dissection or sealing. The body 12 includes a plurality of interconnected segments 14 which enclose a flexible tube 16 as shown in more detail in Figs. 2 and 3.

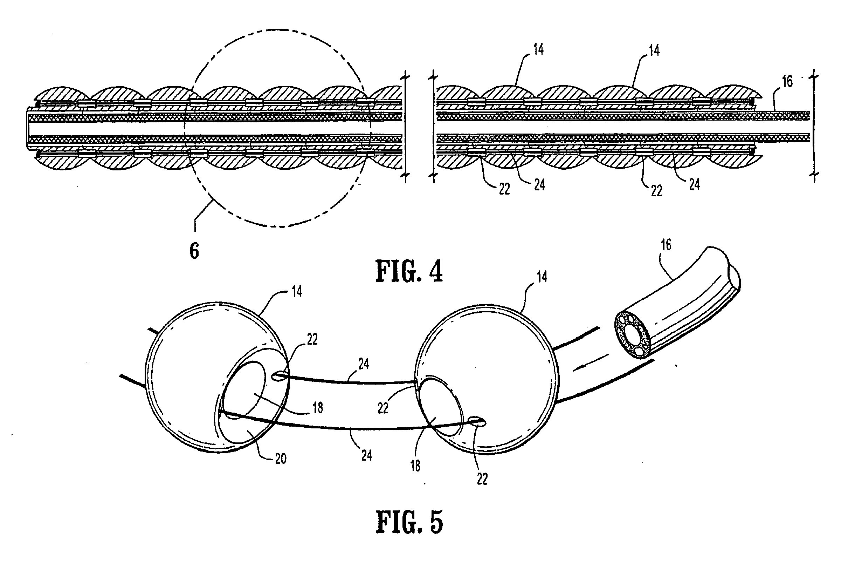

Fig. 2 shows a side cross-sectional view of the segment 14 taken along the line 2. The segment 14 has a generally spherical shape as seen in Fig. 5 and includes a cavity 18, which can be substantially cylindrical, for enclosing the tube 16. Alternately, other cavity configurations are envisioned. With reference to Figs. 4 and 5, each of the segments 14 includes a circular depression 20 (FIG. 5) and two or more openings 22. The circular depression 20 allows the segments 14 to mechanically interface with each other by mating the proximal end of one segment 14 with the depression 20 of the immediately distal segment 14. This allows the segments 14 to pivot relative with respect to each other in a ball-joint fashion. In embodiments, the segments 14 may have a cylindrical shape with the distal and proximal ends retaining semi-spherical form to allow for ball-joint type mating between the segments 14. The segments may be formed from medical grade materials, such as stainless steel, thermoplastics, titanium, or the like.

A tensile element 24 (e.g., tinel, multi-wire cable, etc.) passes through the openings 22 and interconnects the plurality of segments 14 due to predetermined tension in the tensile element 24. The tension in the tensile element 24 is sufficient to maintain the segments 14 in physical contact with each other (e.g., proximal end of one segment 14 with depression 20 of another segment 14). Increasing tension in one of the tensile elements 24 allows for pivoting of the segments 14 in the direction in which tension is being applied. In embodiments, three or more tensile elements 24 may be used to allow for more steering control.

The tube 16 is disposed within the cavity 18 of the segments 14. With reference to Fig. 3, the tube 16 can be formed from a flexible material such as rubber, silicone rubber, and other elastomers. The tube 16 includes an endoscope port 26, one or more auxiliary ports 28 and one or more rigidizing chambers 30 formed therein. The endoscope port 26 may include cables and other actuation mechanisms for manipulating the tool assembly 13. The auxiliary port 28 may be used as a suction port or may include additional instruments, such as lights and a video camera.

The chamber 30 includes a heating element 32 and is filled with fusible material 34. The heating element 32 may be an electrical heating coil, non-electrical heat sources (e.g., heated fluid passage tube, etc.) which heats the material 34. The heating coil may be operable by any type of electrical power source, either a direct or alternating current source. In one embodiment, the chamber 30 may only be filled with the fusible material 34 and does not include the heating element 32, such that the fusible material 34 melts and solidifies in response to external temperatures (e.g., the instrument 10 being inserted into the patient).

The fusible material 34 may be any material which has a melting point at or about a predetermined threshold, such that when the material is heated above the threshold the material 34 is liquefied. In embodiments, the melting point can be any temperature above the body temperature, 37° C, allowing the material 34 to be solid when the material 34 (e.g., instrument 10) is located within the patient. The material 34 may be wax, fusible metal, fats, and the like. Thus, when the material 34 is not heated, the material 34 is solid and the instrument 10 is in rigid form since the solid material 34 prevents pivoting of the segments 14. When the material 34 is heated, the material is liquefied and the instrument 10 becomes flexible allowing for the segments 14 to pivot in the desired direction.

The fusible material 34 may be a fusible alloy containing one or more of the following metals and/or metal alloys, such as bismuth, lead, tin, antimony, indium or cadmium. During transition between liquid and solid phases, these alloys have a relatively small expansion volume allowing for their use within enclosed spaces, such as the chamber 30. When bismuth is alloyed with other metals, such as lead, tin, and cadmium, expansion of the resulting alloy is modified according to the relative percentages of bismuth and other components. Thus, bismuth alloys containing approximately 50 percent of bismuth exhibit little change of volume during solidification. Alloys containing more than this tend to expand during solidification and those containing less tend to shrink during solidification. Exemplary embodiments of the fusible alloys and their melting points are illustrated in Table (1).

**Table (1)**

| | Bismuth | Lead | Tin | Antimony | Indium | Cadmium | Melting Point |
|---|---|---|---|---|---|---|---|
| Alloy 1 | 44.7% | 22.6% | 8.3% | -- | 19.1% | 5.3% | 47°C |
| Alloy 2 | 49% | 18% | 12% | -- | 21% | -- | 58° C |
| Alloy 3 | 50% | 26.7% | 13.3% | -- | -- | 0% | 70° C |
| Alloy 4 | 42.5% | 37.7% | 11.3% | -- | -- | 8.5% | 70-88° C |
| Alloy 5 | 52.5% | 32% | 15.5% | -- | -- | -- | 95° C |
| Alloy 6 | 48% | 28.5% | -- | 9% | -- | 14.5% | 103-227° C |
| Alloy 7 | 55.5% | 44.5% | -- | -- | -- | -- | 124° C |
| Alloy 8 | 58% | 42% | -- | -- | -- | -- | 138° C |
| Alloy 9 | 40% | -- | 60% | -- | -- | -- | 138-170° C |

The chamber 30 also includes one or more coolant tubes 33 disposed therein and surrounded by the fusible material 34. The coolant tubes 33 may be formed from any type of flexible heat-resistant polymer (e.g., polyemide). The coolant tubes 33 of each chamber 30 may be interconnected at the distal end of the instrument 10 so that the coolant tubes 33 are in continuous fluid communication with each other. The coolant tubes 33 are coupled to a source of coolant fluid and a pump (not explicitly shown) which circulate the coolant fluid 35 through the coolant tubes 33. The coolant fluid 35 is supplied to the coolant tubes 33 to cool the material 34 so that the material 34 cools down and rigidizes the instrument 10.

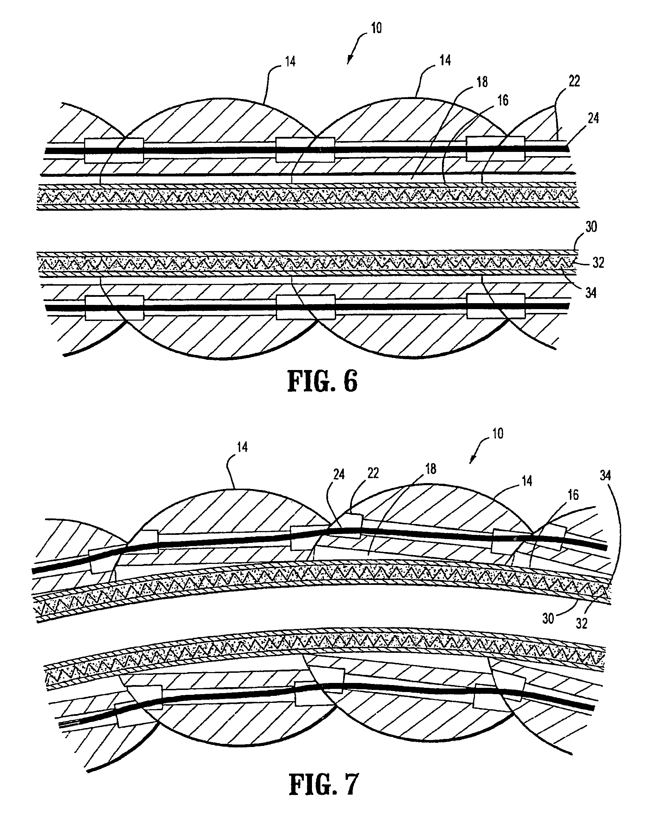

Prior to inserting the instrument 10 into a body lumen, the material 34 inside the chambers 30 is heated above the melting point via the heating elements 32. The heating element 32 is connected to a power source which is controlled by the operator when it is desired to make the instrument 10 flexible. It is envisioned that the power source may include a battery or battery pack positioned near or supported by instrument 10. Once the material 34 is heated so that the material is liquefied, the instrument 10 becomes flexible allowing for the pivoting of the segments 14 and the instrument 10 is guided to the surgical site. Guiding is accomplished by keeping the instrument 10 flexible and steering the instrument 10 by tensioning desired tensile elements 24. Once the instrument 10 is positioned at the site, the instrument 10 is rigidized by decreasing and/or shutting off the heat supplied by the heating element 32 and allowing the material 34 to cool and solidify. In one embodiment, the coolant fluid 35 is supplied to the coolant tubes 33 thereby cooling the material 34 and rigidizing the device 10. The instrument 10 can be rigidized in any desired position, either straight configuration as shown in Fig. 6 or curved configuration as shown in Fig. 7 or in any intermediate configuration. When the instrument 10 needs to be removed or relocated, heat is increased once again to make the instrument 10 flexible and the instrument 10 is guided out of the patient's body.

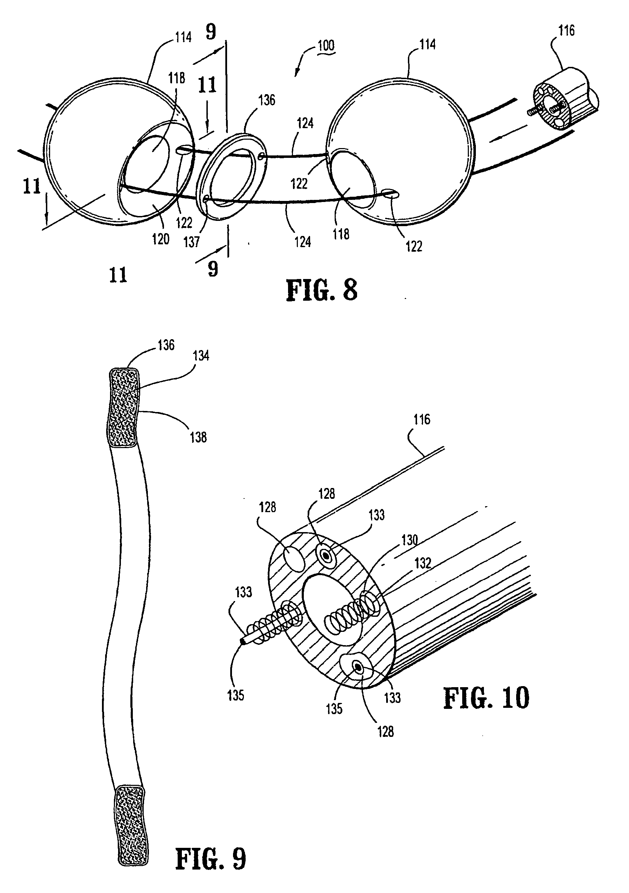

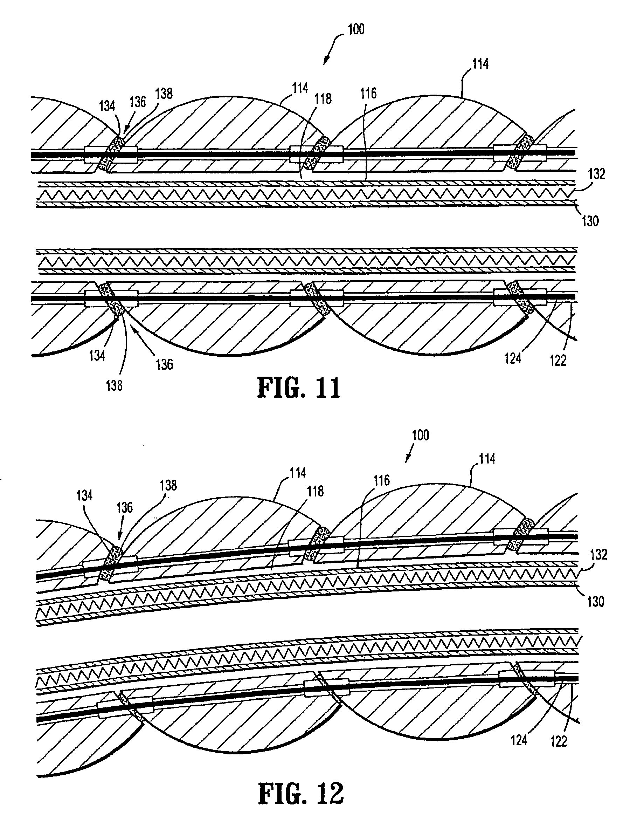

Figs. 8-12 illustrate another embodiment of the flexible steerable instrument 100 which includes a plurality of fittings 136 disposed in between the segments 114. The instrument 100 includes a flexible tube 116 having one or more rigidizing chambers 130 and one or more auxiliary ports 128. The tube 116 is disposed within the segments 114. The fittings 136 include an outer membrane 138 and are filled with the material 134 (Fig. 9). The membrane 138 is formed from a flexible elastomer such that when the material 134 is in liquid form, the fitting 136 can be deformed as shown in Figs. 11 and 12. The fittings 136 include openings 137 which allow for the tensile element 124 to pass therethrough. When assembled, the fittings 136 fit into the depression 122 and are squeezed between the depression 122 and the distal end of the preceding segment 114 when the tensile elements 124 are made taut.

Since the material 134 is in the fittings 136, the chamber 130 includes the heating element 132. The chamber 130 and/or the auxiliary ports 128 may include a coolant tube 133 for supplying coolant fluid 135 through the tube 116. During operation, the instrument 100 is made flexible by increasing the temperature within the chamber 130 which then liquefies the material 134 within the fittings 136. This makes the fittings 136 deformable allowing the segments 114 to pivot and/or swivel relative to each other. The instrument 100 is then guided to the surgical site in flexible form. After the instrument 100 is in the desired position the instrument 100 is rigidized by lowering and/or terminating the heat supplied by the heating elements 132 thereby cooling the material 134 within the fittings 136. This may be accomplished by pumping coolant fluid 135 through the coolant tubes 133. Once the material 134 cools, the fittings 136 become rigid and lock the segments 114 in the desired configuration as shown in Figs. 11 and 12. Fig. 11 shows the instrument 100 rigidized in a straight configuration whereas Fig. 12 shows the instrument 100 in a curved configuration.

The chamber 130 may extend the entire length of the tube 116 or at least one portion thereof. Similarly, the fittings 136 may be disposed between only some of the segments 114 e.g., the distal-most segments, such that only a selected portion or portions of instrument 100 can be rigidized. These arrangements allow for certain portions of the instrument 100 to be rigidized, e.g., tip, while the rest of the instrument 100 can remain flexible.

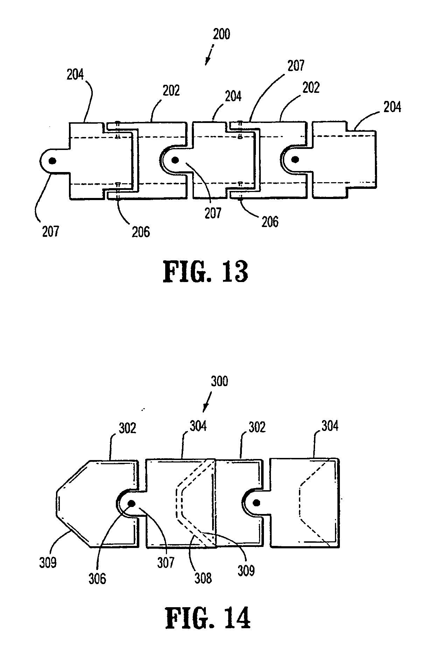

Fig. 13 illustrates another embodiment of the flexible steerable instrument 200. The instrument 200 includes a plurality of pivoting segments namely vertically pivoting segments 202 and horizontally pivoting segments 204. The segments 202 and 204 include one or more hinges 206 which couple the vertically pivoting segments 202 to the horizontally pivoting segments 204. The hinge 206 may include two shafts coupling the segments 202 and 204, each of the shafts connecting a tab 207 to the proximal end of the preceding segment at two points defining a single rotational axis. It is also envisioned that other types of hinges may be used, such as so-called "living hinges."

More specifically, the distal end of the vertically pivoting segment 202 is coupled to the proximal end of the horizontally pivoting segment 204 and the vertically pivoting segment 202 is adapted to hinge in a lateral direction about the hinge 206 thereof with respect to the horizontally pivoting segment 204. The distal end of the horizontally pivoting segment 204 is coupled to the proximal end of the vertically pivoting segment 202 and the horizontally pivoting segment 204 is adapted to hinge in a vertical direction about the hinge 206 thereof with respect to the vertically pivoting segment 202. This configuration provides for interspersing two types of segments having orthogonal hinges such that the consecutive segment rotates in a different direction than the previous segment thereby providing for a flexible steerable instrument 200.

Fig. 14 illustrates a further embodiment of the flexible steerable instrument 300. The instrument 300 includes a plurality of rotational segments 302 separated by pivoting segments 304. Each of the rotational segments 302 is coupled to a pivoting segment 304. The pivoting segments 304 include a hinge 306 and a tab 307 which allows for pivotally coupling the pivoting segments 304 to the proximal end of the rotational segments 302. The pivoting segments 304 also include a circular depression 308 adapted to interface with a hemispherical joint 309 of the rotational segment 302 thereby mechanically interfacing the distal end of the rotational segment 302 with the proximal end of the preceding pivoting segment 304. Consequently, the instrument 300 can be flexed in any desirable direction limited only by the angle of rotation of each of the segments 302 and 304.

The flexible steering instruments 200 and 300 of Figs. 13-14 also include one or more rigidizing chambers having heating elements disposed therein and/or coolant tubes. It is also envisioned that the instruments 300 and 400 may include the rigidizing material either directly within the rigidizing chambers or within the plurality of fittings disposed between the segments as discussed above with respect to Figs. 1-12. In addition, the steering instruments 200 and 300 also include two or more tensile elements for guiding and steering the instruments to the desired location.

The described embodiments of the present disclosure are intended to be illustrative rather than restrictive, and are not intended to represent every embodiment of the present disclosure. Various modifications and variations can be made without departing from the scope of the disclosure as set forth in the following claims. For example, with respect to instrument 10, chamber 30 need not extend the entire length of tube 16 but maybe provided in one or more portions of tube 16, e.g. the distal and central portions of tube 16 or just the distal portion of tube 16.

## Claims

1. A flexible device (10) comprising:
a plurality of interconnected segments (14) configured to pivot relative to each other, each of the plurality of interconnected segments includes a cavity (18) disposed therein; and
a flexible tube (16) disposed within the cavities (18) of the plurality of segments (14), the flexible tube including at least one rigidizing chamber (30) having fusible material (34) disposed therein, wherein upon varying temperature of the fusible material (34), the fusible material (34) shifts between a first state in which the flexible devices (10) is flexible and a second state in which the flexible device (10) is rigidized.

2. The flexible device according to claim 1, further comprising:
a plurality of tensile elements extending through the plurality of interconnected segments wherein tensioning one of the plurality of tensile elements deflects the flexible device.

3. The flexible device according to claim 1, wherein each of the plurality of interconnected segments has a substantially spherical shape and includes a circular depression at a proximal end thereof.

4. The flexible device according to claim 3, wherein each of the plurality of interconnected segments is configured to pivotally fit in the circular depression of a preceding interconnected segment.

5. The flexible device according to claim 1, wherein the flexible tube includes an endoscope port and at least one auxiliary port.

6. The flexible device according to claim 1, wherein the fusible material has a melting point at or above 37°C.

7. The flexible device according to claim 1, wherein the flexible tube is formed from a material selected from rubber, silicone rubber, and elastomer.

8. The flexible device according to claim 1, wherein the at least one rigidizing chamber extends along at least a portion of a length of the flexible tube.

9. The flexible device according to claim 1, wherein the at least one rigidizing chamber further includes at least one heating element configured to vary heat applied to the fusible material.

10. The flexible device according to claim 9, wherein the at least one heating element is selected from the group consisting of electrical heating coil and heated fluid passage tube.

11. The flexible device according to claim 1, wherein the at least one rigidizing chamber further includes at least one coolant tube adapted to supply coolant fluid therethrough and to cool the fusible material.

12. The flexible device according to claim 1, wherein the fusible material is a fusible alloy comprising bismuth and at least one metal selected from the group comprising of lead, tin, antimony, indium or cadmium.

13. The flexible device according to claim 1, wherein the plurality of interconnected segments includes a plurality of vertically pivoting segments and a plurality of horizontally pivoting segments, each of the plurality of vertically pivoting segments being coupled to each of the plurality of the horizontally pivoting segments.

14. The flexible device according to claim 9, a plurality of fittings disposed between each of the plurality of interconnected segments, each of the plurality of fittings including a flexible membrane and fusible material disposed therein.

15. The flexible device according to claim 14, further comprising:
a plurality of tensile elements extending through the plurality of interconnected segments, wherein tensioning one of the plurality of tensile elements deflects the flexible steerable device.

16. The flexible device according to claim 14, wherein each of the plurality of interconnected segments has a substantially spherical shape and includes a circular depression at a proximal end thereof.

17. The flexible device according to claim 16, wherein each of the plurality of interconnected segments is configured to pivotally fit in the circular depression of a preceding interconnected segment.

18. The flexible device according to claim 14, wherein flexible tube includes an endoscope port and at least one auxiliary port or wherein the fusible material has a melting point at or above 37°C or wherein the flexible tube and the plurality of fittings are formed from at material selected from rubber, silicone rubber, and elastomer.

## Patentansprüche

1. Flexible Vorrichtung (10), Folgendes umfassend:
mehrere miteinander verbundene Segmente (14), die dazu ausgelegt sind, sich in Bezug aufeinander zu verschwenken, wobei jedes der mehreren miteinander verbundenen Segmente einen darin vorgesehenen Hohlraum (18) aufweist; und
ein flexibles Rohr (16), das in den Hohlräumen (18) der mehreren Segmente (14) angeordnet ist, wobei das flexible Rohr mindestens eine versteifende Kammer (30) mit darin vorgesehenem, schmelzbarem Material (34) aufweist, wobei bei einer sich verändernden Temperatur des schmelzbaren Materials (34) das schmelzbare Material (34) zwischen einem ersten Zustand, in dem die flexible Vorrichtung (10) flexibel ist, und einem zweiten Zustand wechselt, in dem die flexible Vorrichtung (10) versteift ist.

2. Flexible Vorrichtung nach Anspruch 1, darüber hinaus umfassend:
mehrere dehnbare Elemente, die sich durch die mehreren miteinander verbundenen Segmente erstrecken, wobei eine Dehnung eines der mehreren dehnbaren Elemente die flexible Vorrichtung ablenkt.

3. Flexible Vorrichtung nach Anspruch 1, wobei jedes der mehreren miteinander verbundenen Segmente eine im Wesentlichen kugelförmige Form hat und eine kreisförmige Vertiefung an einem proximalen Ende davon aufweist.

4. Flexible Vorrichtung nach Anspruch 3, wobei jedes der mehreren miteinander verbundenen Segmente dazu ausgelegt ist, sich verschwenkbar in die kreisförmige Vertiefung eines vorhergehenden, damit verbundenen Segments einzupassen.

5. Flexible Vorrichtung nach Anspruch 1, wobei das flexible Rohr eine Endoskopöffnung und mindestens eine zusätzliche Öffnung aufweist.

6. Flexible Vorrichtung nach Anspruch 1, wobei das schmelzbare Material einen Schmelzpunkt hat, der bei oder über 37°C liegt.

7. Flexible Vorrichtung nach Anspruch 1, wobei das flexible Rohr aus einem Material gebildet ist, das aus Kautschuk, Silikonkautschuk und Elastomer ausgewählt ist.

8. Flexible Vorrichtung nach Anspruch 1, wobei sich die mindestens eine versteifende Kammer entlang zumindest eines Abschnitts einer Länge des flexiblen Rohrs erstreckt.

9. Flexible Vorrichtung nach Anspruch 1, wobei die mindestens eine versteifende Kammer darüber hinaus mindestens ein Heizelement aufweist, das dazu ausgelegt ist, eine an das schmelzbare Material angelegte Wärme zu verändern.

10. Flexible Vorrichtung nach Anspruch 9, wobei das mindestens eine Heizelement aus der Gruppe ausgewählt ist, die aus einer elektrischen Heizspule und einem beheizten Flüssigkeitsdurchlaufrohr besteht.

11. Flexible Vorrichtung nach Anspruch 1, wobei die mindestens eine versteifende Kammer darüber hinaus mindestens ein Kühlmittelrohr aufweist, das dazu angepasst ist, Kühlflüssigkeit durch dieses hindurch zuzuführen und das schmelzbare Material zu kühlen.

12. Flexible Vorrichtung nach Anspruch 1, wobei es sich bei dem schmelzbaren Material um eine Schmelzlegierung handelt, die Wismut und mindestens ein Metall enthält, das aus der Gruppe ausgewählt ist, die Blei, Zinn, Antimon, Indium oder Kadmium umfasst.

13. Flexible Vorrichtung nach Anspruch 1, wobei die mehreren miteinander verbundenen Segmente mehrere vertikal verschwenkende Segmente und mehrere horizontal verschwenkende Segmente aufweisen, wobei jedes der mehreren vertikal verschwenkenden Segmente mit jedem der mehreren horizontal verschwenkenden Segmente verbunden ist.

14. Flexible Vorrichtung nach Anspruch 9, wobei mehrere Anschlussvorrichtungen jeweils zwischen den mehreren miteinander verbundenen Segmenten angeordnet sind, wobei jede der mehreren Anschlussvorrichtungen eine flexible Membran und schmelzbares Material aufweist, das darin vorgesehen ist.

15. Flexible Vorrichtung nach Anspruch 14, darüber hinaus umfassend:
mehrere dehnbare Elemente, die sich durch die mehreren miteinander verbundenen Segmente erstrecken, wobei eine Dehnung eines der mehreren dehnbaren Elemente die flexible, lenkbare Vorrichtung ablenkt.

16. Flexible Vorrichtung nach Anspruch 14, wobei jedes der mehreren miteinander verbundenen Segmente eine im Wesentlichen kugelförmige Form hat und eine kreisförmige Vertiefung an einem proximalen Ende davon aufweist.

17. Flexible Vorrichtung nach Anspruch 16, wobei jedes der mehreren miteinander verbundenen Segmente dazu ausgelegt ist, sich verschwenkbar in die kreisförmige Vertiefung eines vorhergehenden, damit verbundenen Segments einzupassen.

18. Flexible Vorrichtung nach Anspruch 14, wobei das flexible Rohr eine Endoskopöffnung und mindestens eine zusätzliche Öffnung aufweist, oder wobei das schmelzbare Material einen Schmelzpunkt hat, der bei oder über 37°C liegt, oder wobei das flexible Rohr und die mehreren Anschlussvorrichtungen aus einem Material gebildet sind, das aus Kautschuk, Silikonkautschuk und Elastomer ausgewählt ist.

## Revendications

1. Dispositif flexible (10) comprenant :
une pluralité de segments interconnectés (14) configurés pour pivoter les uns par rapport aux autres, une cavité (18) étant disposée à l'intérieur de chacun de la pluralité de segments interconnectés ; et
un tube flexible (16) disposé à l'intérieur des cavités (18) de la pluralité de segments (14), le tube flexible comprenant au moins une chambre de rigidification (30) à l'intérieur de laquelle est disposée une matière fusible (34), dans lequel, sous l'effet d'une variation de température de la matière fusible (34), la matière fusible (34) passe d'un premier état dans lequel le dispositif flexible (10) est flexible à un second état dans lequel le dispositif flexible (10) est rigidifié.

2. Dispositif flexible selon la revendication 1, comprenant en outre :
une pluralité d'éléments de traction s'étendant à travers la pluralité de segments interconnectés, dans lequel la mise en tension de l'un des éléments de traction de la pluralité d'éléments de traction entraîne une déflection du dispositif flexible.

3. Dispositif flexible selon la revendication 1, dans lequel chacun de la pluralité de segments interconnectés a une forme sensiblement sphérique et comprend une dépression circulaire au niveau d'une extrémité proximale de celui-ci.

4. Dispositif flexible selon la revendication 3, dans lequel chacun de la pluralité de segments interconnectés est configuré pour s'ajuster de manière pivotante dans la dépression circulaire d'un segment interconnecté précédent.

5. Dispositif flexible selon la revendication 1, dans lequel le tube flexible comprend un port d'endoscope et au moins un port auxiliaire.

6. Dispositif flexible selon la revendication 1, dans lequel la matière fusible a un point de fusion supérieur ou égal à 37°C.

7. Dispositif flexible selon la revendication 1, dans lequel le tube flexible est formé à partir d'une matière sélectionnée parmi le caoutchouc, le caoutchouc silicone et l'élastomère.

8. Dispositif flexible selon la revendication 1, dans lequel la ou les chambres de rigidification s'étendent sur au moins une partie d'une longueur du tube flexible.

9. Dispositif flexible selon la revendication 1, dans lequel la ou les chambres de rigidification comprennent en outre au moins un élément chauffant configuré pour faire varier la chaleur appliquée à la matière fusible.

10. Dispositif flexible selon la revendication 9, dans lequel l'au moins un élément chauffant est sélectionné dans le groupe constitué d'une bobine chauffante électrique et d'un tube de passage de fluide chauffé.

11. Dispositif flexible selon la revendication 1, dans lequel la ou les chambres de rigidification comprennent en outre au moins un tube réfrigérant conçu pour faire circuler un fluide réfrigérant à travers celui-ci et refroidir la matière fusible.

12. Dispositif flexible selon la revendication 1, dans lequel la matière fusible est un alliage fusible comprenant du bismuth et au moins un métal sélectionné dans le groupe comprenant le plomb, l'étain, l'antimoine, l'indium ou le cadmium.

13. Dispositif flexible selon la revendication 1, dans lequel la pluralité de segments interconnectés comprend une pluralité de segments verticalement pivotants et une pluralité de segments horizontalement pivotants, chacun de la pluralité de segments verticalement pivotants étant accouplé à chacun de la pluralité de segments horizontalement pivotants.

14. Dispositif flexible selon la revendication 9, une pluralité de raccords étant disposés entre chacun de la pluralité de segments interconnectés, chacun de la pluralité de raccords comprenant une membrane flexible à l'intérieur de laquelle est disposée une matière fusible.

15. Dispositif flexible selon la revendication 14, comprenant en outre :
une pluralité d'éléments de traction s'étendant à travers la pluralité de segments interconnectés, dans lequel la mise en tension de l'un de la pluralité d'éléments de traction entraîne une déflection du dispositif orientable flexible.

16. Dispositif flexible selon la revendication 14, dans lequel chacun de la pluralité de segments interconnectés a une forme sensiblement sphérique et comprend une dépression circulaire au niveau d'une extrémité proximale de celui-ci.

17. Dispositif flexible selon la revendication 16, dans lequel chacun de la pluralité de segments interconnectés est configuré pour s'adapter de manière pivotante dans la dépression circulaire d'un segment interconnecté précédent.

18. Dispositif flexible selon la revendication 14, dans lequel le tube comprend un port d'endoscope et au moins un port auxiliaire ou dans lequel la matière fusible a un point de fusion supérieur ou égal à 37°C ou dans lequel le tube flexible et la pluralité de raccords sont formés avec un matériau sélectionné parmi le caoutchouc, le caoutchouc silicone et l'élastomère.
